# FASCICULE DE BREVET EUROPEEN

(11) **EP 1 283 708 B1**
(45) Date de publication et mention de la délivrance du brevet: **03.08.2005**
(21) Numéro de dépôt: 01938318.1
(22) Date de dépôt: 23.05.2001
(51) Int. Cl.: A61K 31/155, A61P 17/02

(54) **UTILISATION DE DERIVES DE BIGUANIDE POUR FABRIQUER UN MEDICAMENT AYANT UN EFFET CICATRISANT**
VERWENDUNG VON BIGUANIDEDERIVATEN ZUR HERSTELLUNG EINES VERNARBUNGSFÖRDERNDEN MEDIKAMENTS
USE OF BIGUANIDE DERIVATIVES FOR MAKING A MEDICINE HAVING A WOUND HEALING EFFECT

(30) Priorité: 26.05.2000 FR 0006798
(43) Date de publication de la demande: 19.02.2003
(73) Titulaire: Centre National De La Recherche Scientifique-CNRS, 75794 Paris Cedex 16 (FR)
(72) Inventeur: POTIER, Pierre, Jean-Paul, F-75007 Paris (FR); SASAKI, Nobumichi-André, 80260 Flesselles (FR); ACHAB, Maria, Conception, F-91190 Gif-sur-Yvette (FR); FRANCK, Gisèle, F-94230 Cachan (FR); THAL, Claude, F-92330 Sceaux (FR); BAKALA, Joanna, F-75012 (FR)
(74) Mandataire: Ahner, Francis
(86) Numéro de dépôt international: PCT/FR2001/001598
(87) Numéro de publication internationale: WO 2001/091696

(56) Documents cités:
- EP-A- 0 283 369
- EP-A- 0 535 446
- EP-A- 0 852 148
- WO-A-01/21159
- FR-A- 2 213 778
- FR-A- 2 320 735
- US-A- 3 098 008
- US-A- 3 996 232
- US-A- 4 163 800
- PATENT ABSTRACTS OF JAPAN vol. 1999, no. 01, 29 janvier 1999 (1999-01-29) & JP 10 265391 A (KOBAYASHI PHARMACEUT CO LTD), 6 octobre 1998 (1998-10-06) & DATABASE WPI Week 200033 Derwent Publications Ltd., London, GB; AN 1998-589656 TAKAO KOTA; WAMOTO CHIKASHIGE: "Composition for external wound" & JP 10 265391 A (KOBAYASHI PHARMA), 6 octobre 1998 (1998-10-06)
- DATABASE WPI Week 199050 Derwent Publications Ltd., London, GB; AN 1990-374422 XP002188541 KOLYADENKO V.G. ET AL.: "Method of treating syphilis" & SU 1 560 212 A (KIEV MED INST), 30 avril 1990 (1990-04-30)
- DATABASE WPI Week 199912 Derwent Publications Ltd., London, GB; AN 1999-132924 XP002188542 XIE RUIGANG: "Wound ointment" & CN 1 197 643 A (XIE RUIGANG), 4 novembre 1998 (1998-11-04)
- CUNLIFFE W J: "Dowling oration 1975. Fibrinolysis and vasculitis." CLINICAL AND EXPERIMENTAL DERMATOLOGY, (1976 MAR) 1 (1) 1-16. REF: 70 , XP008000094
- WEIDNER F.: "[Immunologic and inflammatory vascular lesions;clinical features and treatment in dermatology ]. IMMUNOLOGISCHE UND ENTZUNDLICHE GEFASSSCHADEN: KLINIK UND THERAPIE IN DER DERMATOLOGIE." THERAPIEWOCHE, (1979) 29/16 (2769-2779). CODEN: THEWA6, XP000981758
- CAVALERI F. ET AL: "Observations on the use of oral hypoglycaemizing agents in diabetic skin patients, with particular regard to the association glibenclamide- phenformin (Suguan)." GIORNALE ITALIANO DI DERMATOLOGIA / MINERVA DERMATOLOGICA, (1977) 112/3 (255-266). CODEN: GIDMB6, XP000981768
- SHORNICK J.K. ET AL: "Idiopathic atrophie blanche." JOURNAL OF THE AMERICAN ACADEMY OF DERMATOLOGY, (1983) 8/6 (792-798). CODEN: JAADDB, XP000981756

## Description

La présente invention concerne la cicatrisation des plaies. Elle concerne en particulier l'utilisation de dérivés de biguanide ou de leurs sels pharmaceutiquement acceptables, avantageusement la metformine, pour fabriquer un médicament ayant un effet cicatrisant.

La cicatrisation des plaies ou des dommages apparentés de différents types de tissus dépend généralement de la prolifération de nouveaux tissus épithéliaux, endothéliaux et conjonctifs. Elle met donc en jeu une série d'événements coordonnés, cellulaires et moléculaires. Elle peut être retardée ou modifiée par des perturbations métaboliques qui accompagnent certaines maladies de longue durée telles qu'insuffisance veineuse, artérite, diabète et même par certaines thérapies.

Le marché pharmaceutique offre actuellement de nombreuses préparations topiques recommandées pour la cicatrisation des plaies. En fait, leur action résulte de la complémentarité des différents produits qui les composent et qui leur confèrent, dans une certaine limite, leur propriété cicatrisante. Elles protègent les plaies du milieu environnant par un pansement antiseptique. Elles stimulent le développement de la vascularisation et régulent l'épidermisation. Ces formes topiques sont constituées principalement d'un mélange lipidique (lanoline, vaseline, glycérine...) dans lequel sont ajoutés des acides (salicylique, benzoïque, malique), des minéraux (oxyde de zinc, de titane) ou des halogénures (iodure d'amidon).
Certaines contiennent également du collagène, du fibrinogène, du protéolysat enzymatique de sérum (apport d'acides aminés) ou encore des vitamines (vitamine A) ou des hormones (acétate de chloro-4 testostérone). Il existe également une pommade (Madécasol® tulgras des Laboratoires SYNTEX) dont l'action cicatrisante est apportée par l'addition d'un mélange de trois triterpènes extraits de racines de la plante *Centella asiatica* (TCEA). Ces composés exercent leur propriété en stimulant la biosynthèse du collagène et de glycoaminoglycannes. Cependant, ces extraits peuvent provoquer également chez les patients des allergies de contact.

Il est connu qu'une des complications du diabète réside dans l'apparition d'affections cutanées telles que les ulcères (voir angiodermites nécrotiques ulcéreuses) ou de dermatoses perforantes que les médicaments classiques utilisés lors des traitements du diabète ne parviennent ni à contrôler ni à soigner.

Des compositions pharmaceutiques à base de biguanides sont également déjà connues. Mais elles ne sont utilisées que dans le traitement de certaines formes de diabète, et principalement du diabète du type Il non insulino-dépendant, comme agents antihyperglycémiants qui favorisent le retour à l'équilibre glycémique. La metformine est le dérivé de biguanide le plus utilisé dans ce type de traitement.
La posologie journalière est comprise entre 500 mg et 3g suivant le taux de la glycémie du-diabétique. La metformine a une marge thérapeutique élevée chez l'homme et est considérée comme un médicament bien toléré.
L'effet antihyperglycémique de la metformine serait dû d'une part à l'augmentation de l'activité de l'insuline endogène et d'autre part à l'action de la metformine à travers des mécanismes indépendants de l'insuline. En effet, l'action de la metformine se traduit par la diminution de l'absorption intestinale du glucose, l'augmentation de l'absorption cellulaire du glucose sanguin et la diminution de la production du glucose par le foie (suppression de la néoglucogenèse) ainsi que la quantité d'insuline nécessaire pour normaliser la glycémie. Ces effets résultent, en partie, du pouvoir de la metformine à amplifier l'action de l'insuline existante par une augmentation de l'activité de l'enzyme tyrosine kinase du récepteur de l'insuline, ce qui déclenche la cascade de signalisation "post-récepteur".

La demande de brevet FR 2 213 778 décrit de nouvelles compositions destinées au traitement des maladies cutanées proliférantes et pouvant contenir un dérivé de biguanide : la phenformine. Les maladies cutanées proliférantes sont des maladies bénignes et malignes de la peau qui sont caractérisées par une prolifération excessive chronique des cellules de l'épiderme, du derme ou de leur inclusions. Les compositions décrites dans cette demande de brevet réduisent cette prolifération excessive et n'ont donc pas un action cicatrisante, c'est à dire d'accélération de la croissance tissulaire.

Le brevet US A 4 163 800 décrit une composition topique contenant un mélange de composés péroxydés et de dérivés de guanidine ou de la biguanide pour le traitement de lésions cutanées telles que les brûlures ou les ulcères cutanés. Or le peroxyde de benzoïle est utilisé dans ce document comme agent kératolitique et anti-bactérien. C'est donc le péroxyde de benzoïle qui soigne les lésions cutanées par son action kératolitique.

La demande de brevet WO 01/21159 qui est citée au sens de l'article 54(3) CBE décrit des compositions comprenant l'administration de façon simultanée ou séparée dans le temps de la natéglinide ou de la répaglinide et (entre autre) de la metformine pour le traitement des maladies liées au diabète. Toutefois, il s'agit en fait d'une co-administration puisque les deux actifs ont un effet synergique et que la présence de la natéglinide ou de la répaglinide est nécessaire pour obtenir l'effet indiqué.

Or, les inventeurs de la présente invention ont mis en évidence de façon surprenante que des dérivés de biguanide et en particulier la metformine possèdent également de fortes propriétés cicatrisantes, c'est à dire une activité stimulante vis-à-vis d'un phénomène physiologique complexe caractérisé entre autre par une croissance cellulaire accrue au niveau de la plaie. Cette prolifération transitoire se produit en réponse à la perte de l'intégrité cutanée et assure la réparation de tissu profond et la reconstitution de l'épiderme au niveau des plaies.

Ainsi, l'application topique de ce composé sous forme de pommade induit une guérison rapide et de longue durée d'ulcères de jambe chez des sujets diabétiques et la répétition de l'application topique du principe actif renforce cet effet. De plus, la metformine accélère également la cicatrisation des plaies atones chez des sujets non diabétiques.

Compte tenu des difficultés rencontrées pour maîtriser la qualité des produits naturels cicatrisants et du nombre d'étapes fastidieuses nécessaires pour isoler ces composés, les dérivés de biguanide, dont la synthèse est simple, totale et rapide, se présentent comme des principes actifs très intéressants.

La présente invention concerne donc l'utilisation de dérivés de biguanide de formule générale I suivante : 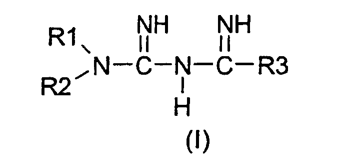 dans laquelle :
les groupes R1 et R2 représentent, indépendamment l'un de l'autre, un atome d'hydrogène, un groupe alkyle en C₁-C₇, un groupe cycloalkyle, un hétérocycle, un groupe alcényle en C₂-C₇, un groupe aryle, un groupe aralkyle, un groupe aryloxylalkyle ou un groupe hétéroaryle,
ou R1 et R2 pris ensemble représentent un alkylène en C₂-C₇ pouvant contenir un ou plusieurs hétéroatomes,
et le groupe R3 représente une amine primaire ou tertiaire,
ou de leurs sels pharmaceutiquement acceptables en tant que seul actif cicatrisant pour fabriquer un médicament destiné à la cicatrisation, ledit médicament étant sous une forme pharmaceutique à usage topique. Avantageusement ce médicament est destiné à être appliqué sur la peau.

Par le terme de « groupe alkyle en C₁-C₇ », on entend au sens de la présente invention tout groupe alkyle en C₁-C₇, linéaire ou ramifié, substitué ou non, comme par exemple les groupes méthyle, éthyle, propyle, isopropyle ou butyle ainsi que leurs isomères.

Par le terme de « groupe cycloalkyle », on entend au sens de la présente invention tout groupe cycloalkyle contenant de 3 à 7 atomes de carbones, comme par exemple le groupe cyclohéxanyle.

Par le terme de « hétérocycle », on entend au sens de la présente invention tout cycle contenant de 3 à 7 atomes, un ou plusieurs d'entre eux étant l'atome d'azote, d'oxygène ou de soufre, les autres étant des atomes de carbones.

Par le terme de « groupe alcényle en C₂-C₇ », on entend au sens de la présente invention tout groupe alcényle en C₂-C₇, linéaire ou ramifié, substitué ou non, tel que les groupes vinyle ou allyle.

Par le terme de « groupe aryle », on entend au sens de la présente invention le groupe phényle, qui peut contenir un ou plusieurs substituants choisis parmi un groupe alkyle en C₁-C₇ tel que défini ci-dessus, un groupe alcényle en C₂-C₇ tel que défini ci-dessus, ou un halogène.

Par le terme de « groupe aralkyle », on entend au sens de la présente invention tout groupe aryle tel que défini ci-dessus lié par l'intermédiaire d'un alkyle tel que défini ci-dessus. Avantageusement, dans le cas où le groupe alkyle représente CH₂, le groupe aryle est un groupe phényle, ce groupe phényle est substitué de la façon définie ci-dessus et dans le cas où le groupe alkyle ne représente pas CH₂, le groupe aryle est tel que défini ci-dessus.

Par le terme de« groupe aryloxyalkyle», on entend au sens de la présente invention tout groupe aryle tel que défini ci-dessus lié par l'intermédiaire d'un groupe oxyalkyle dont le reste alkyle est tel que défini ci-dessus.

Par le terme de « groupe hétéroaryle », on entend au sens de la présente invention tout groupe furyle, isoxazyle, pyridyle, pyrimidyle, et pouvant porter un ou plusieurs substituants, comme par exemple, un groupe alkyle en C₁-C₇ tel que défini ci-dessus, un groupe alcényle en C₂-C₇ tel que défini ci-dessus ou un halogène.

Par le terme de « groupe alkylène en C₂-C₇ », on entend au sens de la présente invention tout groupe alkylène en C₂-C₇ tels que par exemple les groupes éthylène, triméthylène, tétraméthylène ou pentaméthylène.

Par le terme « de sel pharmaceutiquement acceptable », on entend au sens de la présente invention tout sel préparé à partir de tout acide non toxique pharmaceutiquement acceptable, y compris les acides organiques et inorganiques. De tels acides incluent l'acide acétique, benzènesulfonique, benzoïque, citrique, éthanesulfonique, fumarique, gluconique, glutamique, bromhydrique, chlorhydrique, lactique, maléique, malique, mandélique, méthanesulfonique, mucique, nitrique, palmoïque, pantothénique, phosphorique, succinique, tartarique et paratoluènesulfonique. Avantageusement, on utilise l'acide chlorhydrique.

Par le terme de « forme pharmaceutique à usage topique », on entend au sens de la présente invention toute forme pharmaceutique destinée à être appliquée sur la surface de la plaie, en particulier sur la peau ou les muqueuses extérieures ou intérieures, et qui agit localement. En particulier, le médicament peut se présenter sous une forme du type huile, crème, mousse, liniment, lotion, pommade, liquide, gel, lait, poudre ou spray. Les formes peuvent être à véhicule monophasique constituées d'un gel neutre d'hydroxypropylcellulose ou d'un gel chargé formé de carboxyméthylcellulose de sodium. On peut également préparer des crèmes, formes à véhicule biphasique, comportant une phase hydrophile dispersée dans une phase lipophile. Avantageusement, le médicament se présente sous la forme d'un gel ou d'une pommade. De façon avantageuse, le médicament peut se présenter sous la forme d'un pansement actif, ledit pansement étant constitué par un support sur lequel est imprégné ou supporté le ou les dérivés de biguanide, avantageusement sous la forme d'un gel ou d'une pommade. En particulier le pansement actif est constitué par l'association d'un pansement hydrocolloïde et d'un ou des dérivés de biguanide.

Dans un mode de réalisation particulier de l'invention les groupes R1 et R2 représentent, indépendamment l'un de l'autre, un atome d'hydrogène, un groupe alkyle en C₁-C₇, un groupe cycloalkyle, un hétérocycle, un groupe alcényle en C₂-C₇, un groupe aryloxylalkyle ou un groupe hétéroaryle.

Dans un autre mode de réalisation particulier de l'invention, le groupe R3 représente l'amine secondaire de formule suivante :

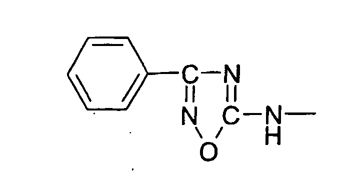

Avantageusement, le dérivé de biguanide utilisé est la metformine, de façon encore plus avantageuse sous la forme d'un chlorhydrate.

Dans un exemple particulier, le médicament contient de 0,02 à 2% en poids du dérivé de biguanide de formule générale I ou de son sel pharmaceutiquement acceptable et un excipient approprié. Ces excipients peuvent être choisis parmi des composés présentant une bonne compatibilité avec ces principes actifs. Il s'agit par exemple des polymères hydrosolubles de type polymère naturel, tels les polysaccharides (gomme xanthane, gomme de caroube, peptine...) ou polypeptides, des dérivés cellulosiques type méthylcellulose, hydroxypropylcellulose, hydroxypropylméthylcellulose ou encore des polymères synthétiques, polaxamers, carbomers, PVA ou PVP.

Enfin, il est à la portée de tout homme de l'art d'ajouter dans ces médicaments divers excipients par exemple des cosolvants comme l'éthanol, le glycérol, l'alcool benzylique, des humectants (glycérol), des agents facilitant la diffusion (transcurol, urée), ou encore des conservateurs anti-bactériens (p-hydroxybenzoate de méthyle à 0,15%).
Dans un mode de réalisation particulier de l'invention, les dérivés de biguanides ou leurs sels pharmaceutiquement acceptables sont combinés avec au moins un autre principe actif. Ce dernier peut être par exemple du type agent antibiotique, antifongique ou antiviral ce qui permet d'accélérer la cicatrisation des tissus endommagés et infectés, simultanément ou en coordination avec le traitement de l'infection sous-jacente.

Les dérivés de biguanide de formule générale I et leurs sels pharmaceutiquement acceptables, en particulier la metformine, avantageusement sous forme de chlorhydrate, peuvent donc améliorer la cicatrisation de tout type de plaies ou lésions. Ces plaies ou lésions peuvent être du type incisions chirurgicales, brûlures thermiques, chimiques ou provoquées par une irradiation, abrasions, lacérations, amputations, ulcères ischémiques ou de décubitus, lésions ou ulcères de la bouche ou les lésions de la cornée, et en particulier celles occasionnées par une intervention chirurgicale réalisée chez des sujets diminués, âgés, traités par radio- ou chimiothérapie, ou diabétiques. Il en est de même de l'ensemble des dermatoses observées chez les malades dont la circulation cutanée est déficiente (lésions érythémateuses, vascularites) et de l'ensemble des plaies observées chez les sujets diabétiques. Les compositions pharmaceutiques et médicaments selon l'invention apparaissent comme bénéfiques également dans la thérapie des nécroses tissulaires, post-thrombotiques par exemple.

Les exemples ci-après de compositions selon l'invention sont donnés à titre d'illustration.

### EXEMPLES

Plusieurs formes pharmaceutiques ont été préparées sans agent conservateur. Les pourcentages sont exprimés en poids.

### Exemple de formulation 1 :

Metformine : 1 % en poids par rapport au poids de gel.
Gel neutre d'hydroxypropylcellulose (Klucel d'Aqualon type 99 MF EP) à 2,9% : complément à 100%.

### Exemple de formulation 2 :

Metformine : 1 % en poids par rapport au poids de gel.
Gel chargé de carboxyméthylcellulose de sodium (Aqualon) à 4,5% : complément à 100%.

### Exemple de formulation 3 :

Metformine : 1 % en poids par rapport à la phase lipophile.
Emulsion d'hydrocérine (excipient gras de chez Roc® contenant de la vaseline, de l'huile de paraffine, des triglycérides, des éthers depolyoxyéthylène et de la cérisine) à 33% (H/L : phase hydrophile dispersée dans une phase lipophile) : complément à 100%.
La préparation de cette émulsion est effectuée à 73°C en versant l'eau dans laquelle la metformine a été solubilisée dans la phase grasse et en agitant jusqu'à refroidissement.

D'autres objets et avantages de l'invention deviendront apparents pour l'homme du métier à partir de la description détaillée ci-dessous et par le biais de référence aux dessins illustratifs suivants.
La figure 1 représente l'effet d'une pommade selon l'exemple de formulation 3 comprenant de la metformine à 1 % en poids par rapport à la phase lipophile sur la cicatrisation cutanée.
La figure 2 représente l'étude « dose-réponse » d'une pommade selon l'exemple de formulation 3 comprenant de la metformine à différentes concentrations sur la cicatrisation cutanée.
La figure 3 représente la surface relative occupée par les trousseaux de collagène dans le tissu de granulation à J4 selon le pourcentage de metformine présent dans la pommade selon l'exemple de formulation 3.

L'efficacité de la metformine a été testée *in vivo* sur des plaies cutanées reproduisant une ulcération. Le pseudo-ulcère a été produit chez des rats Zucker *fa*/*fa* de 14 semaines par perte de matière de forme circulaire de 8 mm de diamètre réalisée par « punch » jusqu'au plancher musculaire. Le traitement quotidien de la plaie par la metformine contenue dans une pommade selon l'exemple de formulation 3 à différentes concentrations de metformine a conduit systématiquement à une amélioration significative de la cicatrisation. La vitesse de la cicatrisation a été évaluée en déterminant la surface des plaies par une vidéo-caméra couplée à un ordinateur équipé d'un logiciel d'analyse d'images.

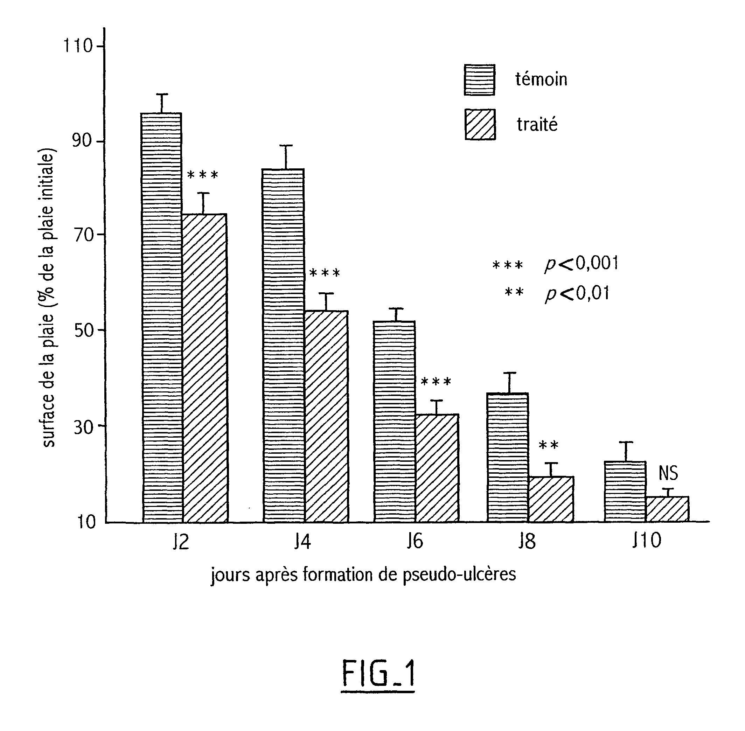

Le traitement des plaies pendant 10 jours (J0-J10) par la pommade selon l'exemple de formulation 3 comprenant de la metformine à la concentration de 1 % en poids par rapport à la phase lipophile a permis de montrer sur le plan macroscopique une diminution importante de la surface des plaies traitées par rapport aux plaies contrôles (Fig. 1). L'évaluation de la cicatrisation sur le plan microscopique à J5 et J10 indique une augmentation de la qualité du tissu cicatriciel dans les plaies traitées avec la pommade selon l'exemple de formulation 3 comprenant de la metformine (tissu de granulation mieux développé, réépidermisation plus avancée). De plus, la metformine à la concentration de 1 % en poids par rapport à la phase lipophile semble également exercer un effet sur la néovascularisation. Cet effet se manifeste à J5 à travers une densité de capillaires sanguins plus importante sur les bords de la plaie des rats traités par rapport aux rats témoins.

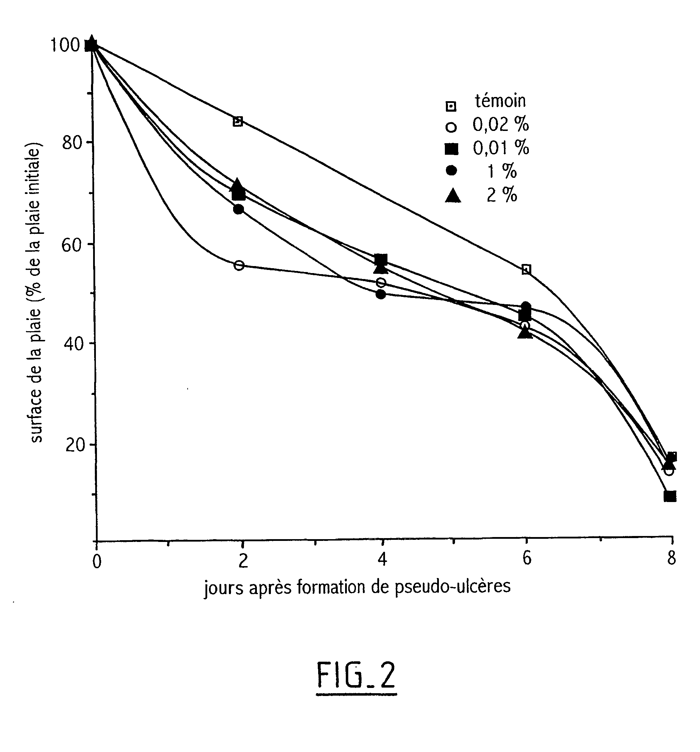

Les résultats d'une étude comparative « dose-réponse » réalisée avec une pommade selon l'exemple de formulation 3 comprenant de la metformine aux concentrations 0,02%, 0,1% , 1% et 2% en poids par rapport à la phase lipophile révèlent un effet cicatrisant pour toutes les concentrations testées. Pour les quatre concentrations de metformine utilisées, la fermeture des plaies traitées est plus rapide par rapport aux plaies témoins non traitées (Fig. 2).
Les résultats de l'analyse microscopique des coupes histologiques des plaies sont rassemblés dans le tableau 1 suivant.

**Tableau 1.**

| **Effet de différentes concentrations de metformine en poids par rapport à la phase lipophile dans une pommade selon l'exemple de formulation 3 sur la formation de l'épiderme (biopsie prélevée au tiers de la plaie)** | | | | |
|---|---|---|---|---|
| **Témoin** | **J4** | **J6** | **J8** | **J10** |
| 1 | - | - | ± | + |
| 2 | - | - | + | + |
| 3 | - | - | + | + |
| 4 | - | ± | + | + |
| 5 | - | + | + | + |
| 6 | - | + | + | + |
| Moyenne relative | 0 % | 50 % | 100 % | 100 % |
| | | | | |
| **Pommade comprenant 0,02 % de metformine** | **J4** | **J6** | **J8** | **J10** |
| 1 | ± | + | + | + |
| 2 | - | + | + | + |
| 3 | + | + | + | + |
| 4 | - | + | + | + |
| Moyenne relative | 50 % | 100 % | 100 % | 100 % |
| **Pommade comprenant 0,1 % de metformine** | **J4** | **J6** | **J8** | **J10** |
| 1 | - | + | + | + |
| 2 | - | ± | + | + |
| 3 | - | + | + | + |
| 4 | - | + | + | + |
| Moyenne relative | 0 % | 100 % | 100 % | 100 % |
| | | | | |
| **Pommade comprenant 1 % de metformine** | **J4** | **J6** | **J8** | **J10** |
| 1 | - | + | + | + |
| 2 | - | ± | + | + |
| 3 | ± | ± | + | + |
| 4 | - | + | + | + |
| Moyenne relative | 25 % | 100 % | 100 % | 100 % |
| **Pommade comprenant 2 % de metformine** | **J4** | **J6** | **J8** | **J10** |
| 1 | - | + | + | + |
| 2 | - | - | - | + |
| 3 | - | + | + | + |
| 4 | ± | ± | ± | + |
| Moyenne relative | 25 % | 75 % | 75 % | 100 % |
| - : absence d'épiderme | | | | |
| + : présence d'épiderme | | | | |
| ± : présence d'épiderme sur une partie de la biopsie | | | | |

Ces résultats indiquent clairement une accélération de l'épidermisation chez les animaux traités par la metformine aux différentes concentrations par rapport aux animaux témoins non traités.

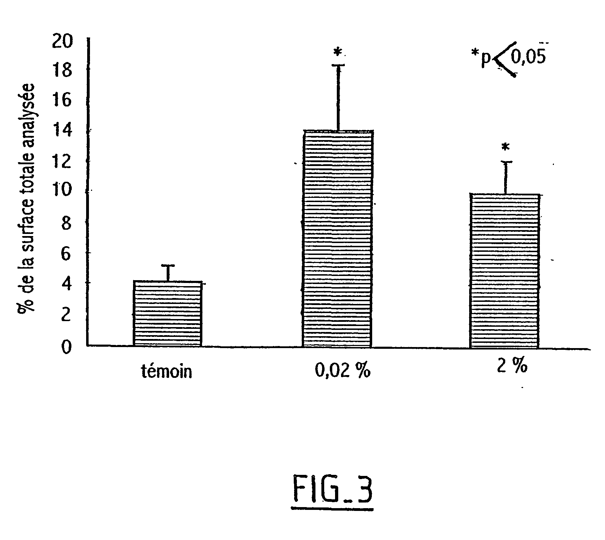

D'autre part, le traitement des plaies avec la metformine augmente significativement la vitesse de maturation du tissu de granulation, ce qui se traduit entre autres par une augmentation de la densité de collagène (Fig.3).

## Revendications

1. Utilisation d'un dérivé de biguanide de formule générale I suivante : 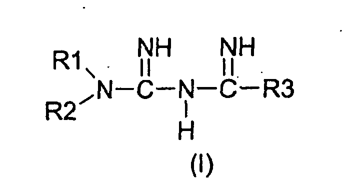 dans laquelle :
les groupes R1 et R2 représentent, indépendamment l'un de l'autre, un atome d'hydrogène ; un groupe alkyle en C₁-C₇ ; un groupe cycloalkyle en C₃-C₇; un hétérocycle comprenant 3 à 7 atomes, un ou plusieurs d'entre eux étant un atome d'oxygène, d'azote ou de soufre, les autres étant des atomes de carbone ; un groupe alcényle en C₂-C₇; un groupe phényle éventuellement substitué par un groupe alkyle en C₁-C₇, un groupe alcényle en C₂-C₇ ou un halogène ; un groupe phénylalkyle dont le groupe alkyle est en C₁-C₇, et le groupe phényle est éventuellement substitué par un groupe alkyle en C₁-C₇, un groupe alcényle en C₂-C₇ ou un halogène ; un groupe phényloxylalkyle dont le groupe alkyle est en C₁-C₇, et le groupe phényle est éventuellement substitué par un groupe alkyle en C₁-C₇, un groupe alcényle en C₂-C₇ ou un halogène ; un groupe furyle, isoxazyle, pyridyle ou pyrimidyle ;
ou R1 et R2 pris ensemble représentent un alkylène en C₂-C₇ pouvant contenir un ou plusieurs hétéroatomes ;
et le groupe R3 représente une amine primaire ou tertiaire ou une amine de formule : 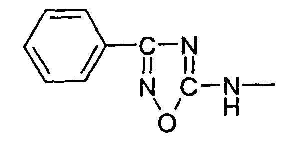 ou de son sel pharmaceutiquement acceptable, en tant que seul actif cicatrisant, pour fabriquer un médicament destiné à la cicatrisation, ledit médicament étant sous une forme pharmaceutique à usage topique.

2. Utilisation selon la revendication 1 pour fabriquer un médicament destiné à la cicatrisation sous une forme pharmaceutique à usage topique destinée à être appliquée sur la peau, avantageusement sous la forme de pommade, éventuellement supporté ou imprégné sur un pansement de façon à constituer un pansement actif.

3. Utilisation selon l'une quelconque des revendications précédentes pour fabriquer un médicament destiné à la cicatrisation des plaies des sujets diabétiques.

4. Utilisation selon l'une quelconque des revendications précédentes **caractérisée en ce que** le dérivé de biguanide est la metformine, avantageusement sous la forme d'un chlorhydrate.

## Patentansprüche

1. Verwendung eines Biguanid-Derivats der folgenden allgemeinen Formel I: 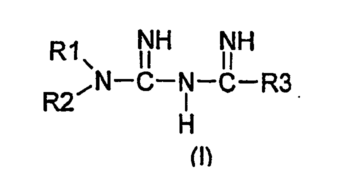 in der:
die Gruppen R1 und R2 unabhängig voneinander ein Wasserstoffatom; eine (C₁-C₇)-Alkylgruppe; eine (C₃-C₇)-Cycloalkylgruppe; einen Heterocyclus, der 3 bis 7 Atome umfasst, wobei ein oder mehrere davon ein Sauerstoff-, Stickstoff- oder Schwefelatom sind, die anderen Kohlenstoffatome sind; eine (C₂-C₇)-Alkenylgruppe; eine Phenylgruppe, die gegebenenfalls mit einer (C₁-C₇)-Alkylgruppe, einer (C₂-C₇)-Alkenylgruppe oder einem Halogen substituiert ist; eine Phenylalkylgruppe, die eine (C₁-C₇)-Alkylgruppe aufweist und deren Phenylgruppe gegebenenfalls durch eine (C₁-C₇)-Alkylgruppe, eine (C₂-C₇)-Alkenylgruppe oder ein Halogen substituiert ist; eine Phenyloxylalkylgruppe, die eine (C₁-C₇)-Alkylgruppe aufweist und deren Phenylgruppe gegebenenfalls durch eine (C₁-C₇)-Alkylgruppe, eine (C₂-C₇)-Alkenylgruppe oder ein Halogen substituiert ist; eine Furyl-, Isoxazyl-, Pyridyl- oder Pyrimidylgruppe darstellen;
oder R1 und R2 zusammengenommen ein (C₂-C₇)-Alkenyl darstellen, das ein oder mehrere Heteroatome enthalten kann;
und die Gruppe R3 ein primäres oder tertiäres Amin oder ein Amin der Formel: 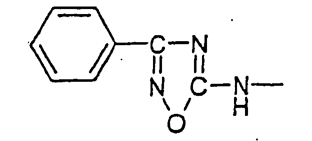 darstellt,
oder seines pharmazeutisch annehmbaren Salzes als einziges aktives die Vernarbungsbildung förderndes Mittel zur Herstellung eines Medikaments, das zur Vernarbung bestimmt ist, wobei das Medikament in einer pharmazeutischen Form zur topischen Verwendung vorliegt.

2. Verwendung nach Anspruch 1 zur Herstellung eines Medikaments, das zur Vernarbung bestimmt ist, in einer pharmazeutischen Form zur topischen Verwendung, die dazu bestimmt ist, auf die Haut aufgetragen zu werden, vorteilhaft in Form einer Salbe, gegebenenfalls getragen oder imprägniert auf einem Verband auf solche Weise, dass ein aktiver Verband gebildet wird.

3. Verwendung nach irgendeinem der vorangehenden Ansprüche zur Herstellung eines Medikaments, das zur Vernarbung von Wunden von diabetischen Subjekten bestimmt ist.

4. Verwendung nach irgendeinem der vorangehenden Ansprüche, **dadurch gekennzeichnet, dass** das Biguanid-Derivat Metformin ist, vorteilhaft in Form eines Hydrochlorids.

## Claims

1. Use of a biguanide derivative of general formula I below: 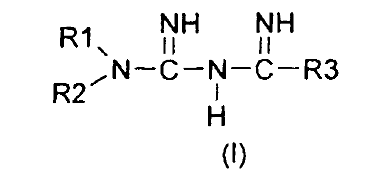 in which:
the groups R1 and R2 represent, independently of each other, a hydrogen atom; a C₁-C₇ alkyl group; a C₃-C₇ cycloalkyl group; a heterocycle containing 3 to 7 atoms, of which one or more are an oxygen, nitrogen or sulfur atom, and the others are carbon atoms; a C₂-C₇ alkenyl group; a phenyl group optionally substituted with a C₁-C₇ alkyl group, a C₂-C₇ alkenyl group or a halogen; a phenylalkyl group the alkyl group of which is C₁-C₇, and the phenyl group is optionally substituted with a C₁-C₇ alkyl group, a C₂-C₇ alkenyl group or a halogen; a phenyloxyalkyl group the alkyl group of which is C₁-C₇ and the phenyl group is optionally substituted with a C₁-C₇ alkyl group, a C₂-C₇ alkenyl group or a halogen;
a furyl, isoxazyl, pyridyl or pyrimidyl group;
or R1 and R2, taken together, represent a C₂-C₇ alkylene which may contain one or more hetero atoms,
and the group R3 represents a primary or tertiary amine, or an amine of formula: 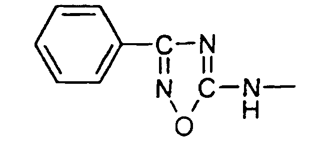 or the pharmaceutically acceptable salt thereof, as the only cicatrizing active agent, to manufacture a medicinal product for cicatrization, the said medicinal product being in a pharmaceutical form for topical use.

2. Use according to Claim 1, to manufacture a medicinal product for cicatrization, in a pharmaceutical form for topical use intended to be applied to the skin, advantageously in the form of an ointment, optionally supported or impregnated on a dressing so as to constitute an active dressing.

3. Use according to either of the preceding claims, to manufacture a medicinal product for cicatrization on the wounds of diabetic individuals.

4. Use according to any one of the preceding claims, **characterized in that** the biguanide derivative used is metformin, advantageously in the form of a hydrochloride.
